# DEMANDE DE BREVET EUROPEEN

(11) **EP 1 093 827 A2**
(43) Date de publication de la demande: **25.04.2001**
(21) Numéro de dépôt: 00402914.6
(22) Date de dépôt: 20.10.2000
(51) Int. Cl.: A61M 35/00, A45D 40/00, A45D 34/04, A61K 7/00

(54) **Applicateur en mousse comprenant un ou plusieurs composés particulaires concentrés au voisinage de la surface d'application**

(30) Priorité: 22.10.1999 FR 9913230
(71) Demandeur: L'OREAL, 75008 Paris (FR)
(72) Inventeur: Vayrette, Sophie, 75015 Paris (FR)
(74) Mandataire: Tanty, François

(57) **Abrégé**

Applicateur comportant un élément d'application (2) réalisé dans une mousse et un élément de protection amovible (3) recouvrant une surface d'application de l'élément d'application, ce dernier comprenant un ou plusieurs composés particulaires (11) destinés à être libérés, après enlèvement de l'élément de protection, notamment pour être mis en contact avec une surface à traiter ou à maquiller ou pour libérer un ou plusieurs produits au contact de ladite surface, caractérisé par le fait que le ou les composés particulaires (11) sont concentrés dans une strate sensiblement parallèle à la surface d'application et adjacente à cette dernière.

## Description

La présente invention concerne un applicateur comprenant un produit cosmétique, dermatologique ou pharmaceutique contenu dans une mousse de matière plastique.

La demande internationale WO 98/16207 décrit un applicateur comprenant des microcapsules dispersées dans une mousse de polyuréthanne.

Cet applicateur comporte une partie amovible adhérant à la mousse, pourvue d'une languette de préhension.

Avant l'utilisation, l'utilisateur malaxe la mousse à travers la partie amovible afin de briser les microcapsules et libérer un actif contenu à l'intérieur, puis retire la partie amovible au moyen de la languette précitée pour dégager la surface d'application.

Les microcapsules sont mélangées lors de la fabrication avec les composants destinés à former la mousse et sont réparties avec un gradient de taille ou de concentration sur toute la hauteur de l'applicateur.

Il en résulte que le produit contenu dans les microcapsules les plus éloignées de la surface d'application peut ne pas atteindre la surface d'application et être perdu.

Il existe un besoin pour disposer d'un applicateur de produit cosmétique, dermatologique ou pharmaceutique qui soit d'utilisation aisée et puisse être réalisé à un coût compatible avec un usage unique.

Il existe également un besoin pour disposer d'un applicateur permettant d'éviter qu'une partie non négligeable du ou des actifs contenus dans l'applicateur soit perdue, faute de gagner la surface à traiter ou à maquiller lors de l'utilisation.

Le nouvel applicateur selon l'invention est du type comportant un élément d'application réalisé dans une mousse et un élément de protection amovible recouvrant une surface d'application de l'élément d'application, ce dernier comprenant un ou plusieurs composés particulaires destinés à être libérés, après enlèvement de l'élément de protection, notamment pour être mis en contact avec une surface à traiter ou à maquiller ou pour libérer un ou plusieurs produits au contact de ladite surface.

Cet applicateur se caractérise par le fait que le ou les composés particulaires sont concentrés dans une strate sensiblement parallèle à la surface d'application et adjacente à cette dernière.

Dans une réalisation préférée, le ou les composés particulaires comprennent des microcapsules renfermant un ou plusieurs actifs.

Grâce à l'invention, le ou les composés particulaires sont libérés ou présents majoritairement au moment de l'utilisation dans le voisinage immédiat de la surface d'application, et sont donc susceptibles d'être utilisés en totalité.

Dans une réalisation préférée, l'élément de protection est réalisé d'un seul tenant par moulage en mousse avec l'élément d'application, l'élément de protection et l'élément d'application étant délimités par une zone de séparation, la surface d'application se situant au niveau de ladite zone de séparation.

Dans ce cas, l'applicateur peut être réalisé à un faible coût, sans utilisation d'adhésif pour maintenir l'élément de protection sur l'élément d'application et sans fabrication séparée de l'élément de protection.

Avantageusement, lorsque le ou les composés particulaires comportent des microcapsules, l'enlèvement de l'élément de protection, qui s'effectue par accrochage ou de préférence par torsion, provoque la rupture des microcapsules et la libération du ou des actifs qu'elles contiennent.

Dans une réalisation préférée, l'applicateur comporte, au niveau de la zone de séparation, une amorce de déchirure.

Avantageusement, l'élément de protection comporte un renfoncement dans l'axe de l'applicateur, permettant d'obtenir lors de la fabrication de l'applicateur un chemin d'expansion préférentiel de la mousse, de manière à ce que le ou les composés particulaires se concentrent à proximité de la zone de séparation, de préférence selon une strate perpendiculaire à l'axe de l'applicateur.

Dans une réalisation particulière, le renfoncement précité est sensiblement hémisphérique, ouvert dans la direction opposée à l'élément d'application.

Le diamètre moyen des particules du ou des composés particulaires est compris par exemple entre 100 µm et 1 mm, la mousse ayant de préférence des cellules de même taille que ces particules.

Avantageusement, l'applicateur est recouvert à sa surface d'une peau, ce qui améliore son aspect et sa tenue mécanique.

Cette peau peut constituer une barrière sensiblement étanche et permet d'éviter, notamment lorsque l'applicateur comporte des microcapsules, à l'utilisateur d'avoir ses doigts au contact du ou des actifs qu'elles renferment, après rupture de ces dernières.

La peau peut également contribuer à la conservation à l'abri de l'air du ou des composés particulaires contenus dans l'applicateur.

Les microcapsules peuvent contenir un ou plusieurs produits actifs devant être conditionnés séparément et être mélangés extemporanément.

L'invention a encore pour objet un procédé pour fabriquer un applicateur comprenant un élément d'application en mousse comportant un ou plusieurs composés particulaires tels que des microcapsules, et un élément de protection amovible recouvrant une surface d'application de l'élément d'application, caractérisé par le fait qu'il comporte les étapes consistant à :
- injecter dans un moule une quantité initiale de mousse,
- déposer sur la mousse précédemment injectée le ou lesdits composés particulaires, la géométrie du moule étant choisie de manière à créer un chemin d'expansion préférentiel de la mousse, de façon à amener la majorité du ou des composés particulaires dans une région de l'élément d'application adjacente à la surface d'application.

Avantageusement, on injecte une quantité initiale de mousse dans le moule, puis avant la fin de l'expansion de la mousse on dépose le ou les composés particulaires, que l'on recouvre ensuite d'une quantité finale de mousse.

De préférence, la quantité initiale de mousse est injectée de manière à remplir partiellement la partie inférieure du moule, destinée au moulage de l'élément d'application.

Toujours de préférence, la partie inférieure du moule présente un profil s'évasant vers le haut, ce qui confère une forme ergonomique à l'élément d'application.

Le moule comporte avantageusement un filet annulaire au niveau de la zone de séparation entre l'élément d'application et l'élément de protection, pour former une amorce de déchirure.

La paroi supérieure du moule présente avantageusement un renfoncement dans l'axe du moule, afin de créer un chemin d'expansion préférentiel de la mousse.

Dans une réalisation particulière, le renfoncement précité est concave vers l'extérieur, de préférence sensiblement hémisphérique.

De préférence, la distance mesurée dans l'axe du moule entre la zone de séparation et la paroi supérieure du moule est au moins deux fois plus faible au centre du moule qu'à sa périphérie.

De préférence encore, la paroi du moule est chauffée de manière à former une peau.

L'invention a encore pour objet l'utilisation d'un applicateur tel que défini plus haut pour l'application d'un produit cosmétique, pharmaceutique ou dermatologique sur la peau, les cheveux, les lèvres ou les ongles.

L'invention a encore pour objet une machine pour la fabrication d'un applicateur en mousse comprenant un ou plusieurs composés particulaires et une surface d'application recouverte par un élément de protection amovible, caractérisée par le fait qu'elle comporte :
- au moins un moule,
- des moyens d'injection pour l'injection de mousse dans ce moule,
- des moyens pour déposer un ou plusieurs composés particulaires dans le moule après une injection de mousse, l'applicateur obtenu comportant un élément d'application dans lequel le ou les composés particulaires sont concentrés dans une strate sensiblement parallèle à la surface d'application et adjacente à cette dernière.

De préférence, les moyens d'injection sont agencés pour réaliser l'injection discontinue de mousse dans le moule, le ou les composés particulaires étant déposés dans le moule entre deux injection de mousse.

D'autres caractéristiques et avantages de la présente invention ressortiront à la lecture de la description détaillée qui va suivre, d'un exemple de réalisation non limitatif, et à l'examen du dessin annexé sur lequel :
- la figure 1 est une vue schématique en perspective d'un applicateur conforme à l'invention,
- la figure 2 est une vue en coupe axiale de l'applicateur de la figure 1,
- la figure 3 est une vue de côté selon la flèche III de la figure 1,
- la figure 4 est une vue arrière selon la flèche IV de la figure 1,
- les figures 5 à 7 illustrent différentes étapes de la fabrication de l'applicateur.

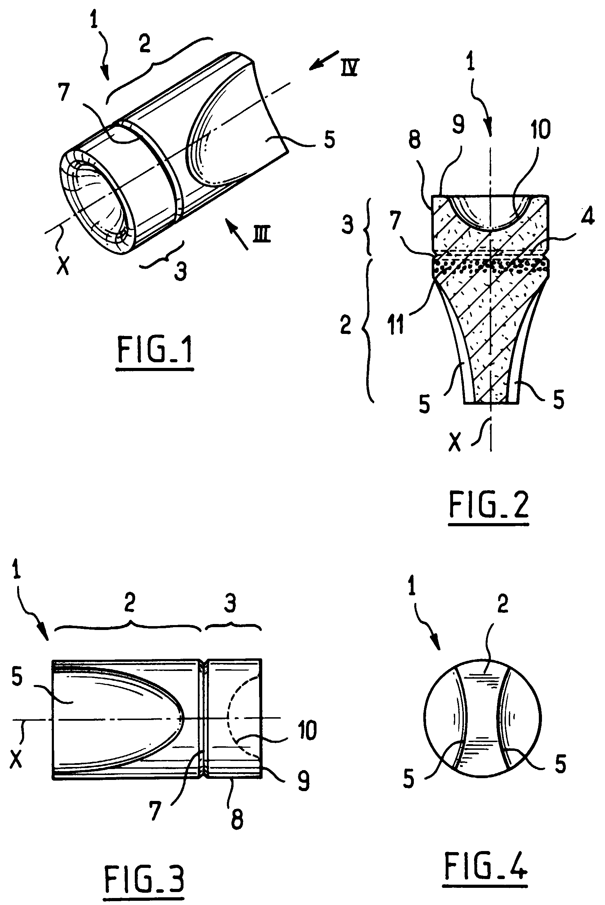

L'applicateur 1 représenté sur les figures 1 à 4, d'axe X, comporte un élément d'application 2 constituant également une partie de préhension et un élément de protection amovible 3, réalisés d'un seul tenant en mousse et délimités par une zone de séparation 4 sensiblement plane et perpendiculaire à l'axe X.

L'élément d'application 2 présente une forme générale s'évasant vers le haut, comme on peut le voir sur la figure 2, avec deux faces principales opposées 5 présentant des renfoncements concaves vers l'extérieur, destinés à recevoir les extrémités des doigts de l'utilisateur.

L'applicateur 1 comporte sur sa surface extérieure, au niveau de la zone de séparation 4, un rétreint annulaire 7 créant une amorce de déchirure.

L'élément de protection 3 comporte une surface latérale 8 cylindrique de révolution autour de l'axe X et une face supérieure 9, plane et perpendiculaire à l'axe X au niveau de son raccordement à la surface latérale 8 et pourvue en son centre d'un renfoncement 10 sensiblement hémisphérique, dont le rôle sera expliqué dans la suite.

L'applicateur 1 comporte, noyée dans l'élément d'application 2 au voisinage de la zone de séparation 4, une strate de composés particulaires, ici des microcapsules 11 contenant un produit cosmétique, dermatologique ou pharmaceutique.

Les microcapsules 11 ont une taille sensiblement homogène dans l'épaisseur de la strate.

Dans l'exemple de réalisation décrit, l'applicateur 1 comporte au niveau de la strate de microcapsules 11 environ 75 % en volume de microcapsules, les 25 % restants étant occupés par la mousse.

La hauteur de la strate de microcapsules 11 est de 2 ou 3 mm par exemple, la hauteur totale de l'applicateur étant comprise entre 20 et 30 mm par exemple.

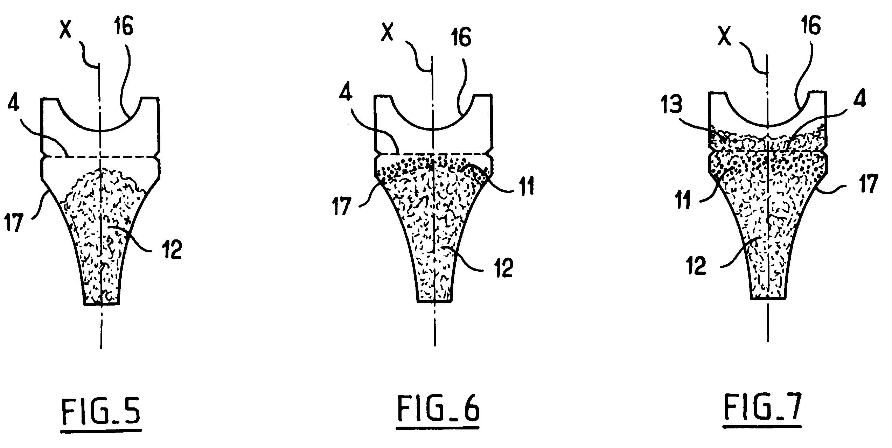

Dans l'exemple de réalisation décrit, l'élément d'application 2 et l'élément de protection 3 sont réalisés dans une mousse de polyuréthanne en mettant en oeuvre le procédé de fabrication qui va maintenant être décrit en référence aux figures 5 à 7.

On commence par injecter dans un moule 17 dont la forme correspond à celle de l'applicateur 1 une quantité initiale 12 de mousse de polyuréthanne.

Cette mousse subit une expansion vers le haut et, sous l'effet notamment du gradient de température dans le moule, avance avec un front présentant un profil arrondi, comme on peut le voir sur la figure 5.

Avant que la mousse n'ait terminé son expansion, on dépose par exemple au moyen d'un jet d'air les microcapsules 11, comme illustré sur la figure 6.

On recouvre ensuite les microcapsules 11 d'une quantité finale 13 de mousse, comme illustré sur la figure 7.

Le profil de la paroi supérieure 16 du moule 17 est choisi de manière à créer un chemin d'expansion préférentiel de la mousse située sous les microcapsules 11 de façon à limiter son expansion au centre du moule dans l'axe X et la favoriser sur les côtés.

En d'autres termes, on contrecarre l'avancée de la quantité initiale 12 de mousse selon l'axe X au bénéfice de sa progression le long des côtés du moule 17, de sorte que le front de la mousse sur lequel reposent les microcapsules 11 tende à s'aplanir et devienne sensiblement parallèle à la zone de séparation 4, au terme de l'expansion de la mousse dans le moule 17.

Ce procédé permet de concentrer les microcapsules 11 dans une strate d'épaisseur réduite à proximité de la zone de séparation, l'épaisseur de cette strate représentant par exemple moins de 20 % de la hauteur totale de l'élément d'application.

Dans l'exemple de réalisation décrit, on utilise une mousse de polyuréthanne non thixotrope, obtenue de façon connue en soi par polycondensation d'un polyol et d'un isocyanate en présence d'eau.

Bien entendu, l'invention n'est pas limitée à l'emploi d'une mousse de polyuréthanne et l'on peut réaliser l'applicateur dans tout autre matériau spongieux déformable à cellules ouvertes ou semi-ouvertes injectable dans un moule.

De préférence, la paroi du moule 17 est chauffée légèrement durant la fabrication de l'applicateur de manière à former à la surface de ce dernier une peau relativement lisse tendant notamment à le rigidifier et à améliorer son aspect.

La mousse utilisée est de préférence choisie de manière à ce que la taille de ses cellules corresponde sensiblement à celle des microcapsules, ces dernières ayant un diamètre extérieur compris de préférence, en moyenne, entre 100 µm et 1 mm.

Le diamètre moyen des microcapsules 11 est par exemple de 500 à 800 µm pour un parfum, une huile, une lotion ou un lait, et de 800 µm à 1 mm pour un produit cosmétique tel qu'un rouge à lèvres ou un fond de teint.

Pour utiliser l'applicateur 1, l'utilisateur saisit entre deux doigts d'une main l'élément d'application 2 et à l'aide de l'autre main tord et arrache l'élément de protection 3 autour de l'axe X.

Cette torsion, d'un quart de tour par exemple, provoque le déchirement de la mousse le long de la zone de séparation 4 et le détachement de l'élément de protection 3, ainsi qu'une rupture des microcapsules concentrées au voisinage de la zone de séparation 4.

La surface de l'élément d'application 2 dégagée par le départ de l'élément de protection 3 constitue une surface d'application destinée à être amenée au contact de la zone à traiter ou à maquiller.

Ainsi, après enlèvement de l'élément de protection 3, l'élément d'application 2 est immédiatement disponible pour l'application du produit.

Autrement dit, il n'est pas nécessaire dans l'exemple décrit, contrairement à ce qui est le cas dans la demande internationale WO 98/16207 précitée, de malaxer au préalable l'applicateur.

Bien entendu, on ne sort pas du cadre de la présente invention lorsque la torsion exercée pour séparer l'élément de protection 3 est insuffisante pour provoquer la rupture des microcapsules et qu'un malaxage de l'élément d'application 2 est nécessaire.

L'applicateur selon l'invention peut être utilisé comme échantillon afin de permettre au public de tester un nouveau produit cosmétique, par exemple un rouge à lèvres ou un fond de teint contenu dans les microcapsules.

L'applicateur selon l'invention peut encore être utilisé par exemple pour l'application topique d'un produit de traitement de l'acné.

Comme matériaux utilisables pour réaliser l'applicateur, on peut citer outre la mousse de polyuréthanne, les mousses d'élastomère de silicone, d'EVA, de polysulfones ou de polyéthylène, cette liste étant non limitative.

L'invention n'est pas limitée à l'exemple de réalisation décrit et à la place des microcapsules, ou en mélange avec ces dernières, on peut utiliser d'autres composés particulaires, comme par exemple des particules d'une substance pulvérulente telle que des microbilles de poudre de maquillage compactée.

## Revendications

1. Applicateur comportant un élément d'application (2) réalisé dans une mousse et un élément de protection amovible (3) recouvrant une surface d'application de l'élément d'application, ce dernier comprenant un ou plusieurs composés particulaires (11) destinés à être libérés, après enlèvement de l'élément de protection, notamment pour être mis en contact avec une surface à traiter ou à maquiller ou pour libérer un ou plusieurs produits au contact de ladite surface, caractérisé par le fait que le ou les composés particulaires (11) sont concentrés dans une strate sensiblement parallèle à la surface d'application et adjacente à cette dernière.

2. Applicateur selon la revendication 1, caractérisé par le fait que le ou les composés particulaires comprennent des microcapsules (11) renfermant un ou plusieurs actifs

3. Applicateur selon la revendication précédente, caractérisé par le fait que les microcapsules renferment un ou plusieurs produits actifs devant être conditionnés séparément et être mélangés extemporanément.

4. Applicateur selon l'une des deux revendications précédentes, caractérisé par le fait que l'enlèvement de l'élément de protection (3), qui s'effectue par arrachage ou de préférence par torsion, provoque la rupture des microcapsules et la libération du ou des actifs qu'elles contiennent.

5. Applicateur selon l'une des revendications précédentes, caractérisé par le fait que l'élément de protection (3) est réalisé d'un seul tenant par moulage en mousse avec l'élément d'application (2), l'élément de protection et l'élément d'application étant délimités par une zone de séparation (4), la surface d'application se situant au niveau de ladite zone de séparation.

6. Applicateur selon la revendication précédente, caractérisé par le fait qu'il comporte une amorce de déchirure au niveau de la zone de séparation (4).

7. Applicateur selon l'une quelconque des revendications précédentes, caractérisé par le fait que l'élément de protection (3) comporte un renfoncement (10) dans l'axe X de l'applicateur, permettant d'obtenir lors de la fabrication de l'applicateur un chemin d'expansion préférentiel de la mousse, de manière à ce que le ou les composés particulaires se concentrent à proximité de la zone de séparation, de préférence selon une strate perpendiculaire à l'axe de l'applicateur.

8. Applicateur selon la revendication précédente, caractérisé par le fait que ledit renfoncement (10) est sensiblement hémisphérique, ouvert dans la direction opposée à l'élément d'application (2).

9. Applicateur selon l'une quelconque des revendications précédentes, caractérisé par le fait que le diamètre moyen du ou des composés particulaires (11) est compris entre 100 µm et 1 mm.

10. Applicateur selon l'une quelconque des revendications précédentes, caractérisé par le fait qu'il est recouvert à sa surface d'une peau.

11. Applicateur selon l'une quelconque des revendications précédentes, caractérisé par le fait qu'il est réalisé dans une mousse de polyuréthanne.

12. Procédé pour fabriquer un applicateur comprenant un élément d'application en mousse comportant un ou plusieurs composés particulaires tels que des microcapsules (11), et un élément de protection amovible (3) recouvrant une surface d'application de l'élément d'application (2), caractérisé par le fait qu'il comporte les étapes consistant à :
- injecter dans un moule (17) une quantité initiale de mousse,
- déposer sur la mousse précédemment injectée le ou lesdits composés particulaires (11), la géométrie du moule étant choisie de manière à créer un chemin d'expansion préférentiel de la mousse, de façon à amener la majorité du ou des composés particulaires dans une région de l'élément d'application adjacente à la surface d'application.

13. Procédé selon la revendication précédente, caractérisé par le fait que l'on injecte une quantité initiale de mousse dans le moule, puis avant la fin de l'expansion de la mousse, on dépose le ou les composés particulaires, que l'on recouvre ensuite d'une quantité finale de mousse

14. Procédé selon la revendication précédente, caractérisé par le fait que la quantité initiale (12) de mousse est injectée de manière à remplir partiellement la partie inférieure du moule (17), destinée au moulage de l'élément d'application (2).

15. Procédé selon la revendication précédente, caractérisé par le fait que la partie inférieure du moule (17) présente un profil s'évasant vers le haut.

16. Procédé selon l'une quelconque des trois revendications précédentes, caractérisé par le fait que le moule (17) comporte un filet annulaire au niveau de la zone de séparation (4) entre l'élément d'application et l'élément de protection pour former une amorce de déchirure (7).

17. Procédé selon l'une quelconque des quatre revendications précédentes, caractérisé par le fait que la paroi supérieure du moule (17) présente un renfoncement (16) dans l'axe (X) du moule.

18. Procédé selon la revendication précédente, caractérisé par le fait que ledit renfoncement (16) est concave vers l'extérieur.

19. Procédé selon la revendication précédente, caractérisé par le fait que ledit renfoncement (16) est sensiblement hémisphérique.

20. Procédé selon l'une quelconque des trois revendications précédentes, caractérisé par le fait que la distance mesurée dans l'axe (X) du moule (17) entre la zone de séparation (4) et la paroi supérieure du moule est au moins deux fois plus faible au centre du moule qu'à sa périphérie.

21. Procédé selon l'une quelconque des revendications 12 à 20, caractérisé par le fait que la paroi du moule est chauffée de manière à former une peau.

22. Procédé selon l'une quelconque des revendications 12 à 21, caractérisé par le fait que la mousse utilisée est choisie dans la liste suivante : mousse de polyuréthanne, de polyéthylène, d'élastomère de silicone, d'EVA, de polysulfones.

23. Procédé selon l'une quelconque des revendications 12 à 22, caractérisé par le fait que la taille moyenne du ou des composés particulaires (11) est comprise entre 100 µm et 1 mm, de préférence entre 500 µm et 800 µm pour des microcapsules renfermant un parfum, une huile, un lait ou une lotion, et entre 800 µm et 1 mm pour des microcapsules renfermant un produit cosmétique tel qu'un rouge à lèvres ou un fond de teint.

24. Utilisation d'un applicateur tel que défini dans l'une quelconque des revendications 1 à 11 pour l'application d'un produit cosmétique, pharmaceutique ou dermatologique sur la peau, les cheveux, les lèvres ou les ongles.

25. Utilisation selon la revendication précédente, l'élément de protection (3) étant séparé de l'élément d'application (2) par un mouvement d'arrachage ou de préférence de torsion autour de l'axe (X) de l'applicateur.

26. Machine pour la fabrication d'un applicateur tel que défini dans l'une quelconque des revendications 1 à 11, caractérisée par le fait qu'elle comporte :
- au moins un moule (17),
- des moyens d'injection pour l'injection de mousse dans ce moule,
- des moyens pour déposer un ou plusieurs composés particulaires (11) dans le moule après une injection de mousse, l'applicateur obtenu comportant un élément d'application (2) dans lequel le ou les composés particulaires sont concentrés dans une strate sensiblement parallèle à la surface d'application et adjacente à cette dernière.

27. Machine selon la revendication précédente, caractérisée par le fait que les moyens d'injection sont agencés pour réaliser l'injection discontinue de mousse dans le moule, le ou les composés particulaires (11) étant déposés dans le moule (17) entre deux injections de mousse.
